# EUROPÄISCHE PATENTSCHRIFT

(11) **EP 1 620 049 B1**
(45) Veröffentlichungstag und Bekanntmachung des Hinweises auf die Patenterteilung: **10.01.2007**
(21) Anmeldenummer: 04730405.0
(22) Anmeldetag: 30.04.2004
(51) Int. Cl.: A61F 9/013

(54) **VORRICHTUNG ZUM SCHNEIDEN DER HORNHAUT EINES AUGES**
DEVICE FOR CUTTING THE CORNEA OF AN EYE
DISPOSITIF POUR DECOUPER LA CORNEE

(30) Priorität: 02.05.2003 AT 6632003; 30.05.2003 AT 8402003; 12.08.2003 AT 12642003; 25.02.2004 AT 2992004
(43) Veröffentlichungstag der Anmeldung: 01.02.2006
(73) Patentinhaber: Daxer, Albert, 4020 Linz (AT)
(72) Erfinder: Daxer, Albert, 4020 Linz (AT)
(74) Vertreter: KLIMENT & HENHAPEL
(86) Internationale Anmeldenummer: PCT/AT2004/000147
(87) Internationale Veröffentlichungsnummer: WO 2004/096106

(56) Entgegenhaltungen:
- DE-A- 3 433 581
- US-A- 6 083 236
- US-B1- 6 506 198

## Beschreibung

### Technisches Gebiet

Die Erfindung bezieht sich auf eine Vorrichtung zum Schneiden der Hornhaut eines Auges zu seiner Brechkraftkorrektur mit einem Gestell, das sowohl einen an das Auge ansaugbaren Ringkörper als auch eine koaxial zum Ringkörper verstellbare Aufnahme für einen Stempel zur Hornhautbeaufschlagung innerhalb des Ringkörpers aufweist, und mit einer auf dem Gestell in einer zum Ringkörper achsnormalen Ebene geführten Halterung für ein das Gestell in einer Umfangsausnehmung durchsetzendes, dem Stempel vorgelagertes Messer, das über die Halterung zum Einschneiden einer lediglich einen tunnelartigen Zugang aufweisenden Tasche in das Hornhautgewebe einerseits radial zum Ringkörper verschiebbar und andererseits um eine zur Führungsebene senkrechte Achse verschwenkbar ist.

### Stand der Technik

Um die Hornhaut eines Auges in seiner Brechkraft korrigieren zu können, ist es bekannt (US 2001/0004702 A1), über einen beispielsweise ca. 4 mm breiten Zugang das Innere des Hornhautgewebes zu behandeln. Dabei wird im Inneren der Hornhaut eine Tasche geschaffen, in die ein Implantat zur Brechkraftkorrektur eingebracht werden kann. Eine Behandlung im Inneren der Hornhaut über einen tunnelartigen Zugang hat gegenüber anderen Verfahren zur Brechkraftkorrektur, bei denen das Hornhautgewebe entweder oberflächlich abgetragen (DE 34 33 581 A1) oder auch die Hornhaut zum Ausbilden einer wegklappbaren Kappe weit eingeschnitten (LASIK Methode) wird, den Vorteil, daß kaum postoperative Schmerzen auftreten bzw. die Stabilität der Hornhaut kaum beeinträchtigt wird, da die für die Stabilität der Hornhaut wichtige obere Schicht, darunter fällt beispielsweise die Bowmansche Membran, größtenteils unverletzt bleibt. Eine Behandlung der Hornhaut über einen tunnelartigen Zugang bietet daher gegenüber den anderen Behandlungsarten eine Vielzahl von Vorteilen. Um solch eine Brechkraftkorrektur durchführen zu können, schlägt die US 2001/0004702 A1 vor, ein Gestell am Auge eines Patienten zu befestigen, an der eine Halterung für ein bewegbares Messer befestigt ist. Das Messer durchsetzt das Gestell in einer Umfangsausnehmung, und wird über eine Halterung in einer Führungsebene geführt. Diese Führungsebene liegt parallel zu Schnittebene des Messers, in welcher Schnittebene eine Tasche in der Hornhaut erstellt wird. Außerdem wird das Messer in seiner Schnittebene am Gestell aufliegend mit einer Feder niedergehalten, da ein Abrutschen des Messers von seinem Ansatz an der Hornhaut zum Erstellen des tunnelartigen Zugangs vermieden werden muß. Es ist nämlich vergleichsweise schwierig, die elastische und äußerst feste äußere Schicht der Hornhaut zu durchdringen. Außerdem darf sich das Messer beim Schneiden nicht verbiegen, da sonst eine exakte Schnittebene und die Vermeidung einer Perforation der oberen oder unteren Schicht der Hornhaut nicht garantiert werden kann, was einer erfolgreichen Brechkraftkorrektur entgegensteht. Zwar ist mit Hilfe von Messern mit hoher Härte (beispielsweise aus einem Diamantmaterial) dem entgegen zusteuern, derartige Messer sind jedoch besonders empfindlich auf Schubbeanspruchungen, was eine Verwendung derartiger Messer bei der Vorrichtung gemäß der US 2001/0004702 A1 aufgrund der Auflage am Gestell ausschließt. Außerdem würde bei solchen Messern neben ihrer Bruchgefährlichkeit einer hohe Beeinträchtigung der Schärfe auftreten.

Desweiteren ist es aus dem Stand der Technik bekannt (LASIK, US 5 556 406 A), nach einem breitflächigen Einschneiden des Hornhautgewebes die entstehende Kappe weg zuklappen und mit einem Laser das frei liegende Hornhautgewebe durch Gewebsabtragung zu behandeln, oder auch dort eine Kunstlinse aufzubringen, bevor die Kappe dann wieder zurück geklappt wird. Nachteilig bei diesem Verfahren ist, daß, wie bereits erwähnt, dabei die Stabilität der Hornhaut stark in Mitleidenschaft gezogen wird, was mitunter zu unerwünschten Verwölbungen der Hornhautoberfläche führen kann.

Die Patentschrift DE-A-3433581 offenbart eine Vorrichtung zum Schneiden der Hornhaut gemäß dem Oberbegriff des Anspruchs 1.

### Darstellung der Erfindung

Der Erfindung liegt somit die Aufgabe zugrunde, ausgehend vom eingangs geschilderten Stand der Technik eine Vorrichtung zum Schneiden der Hornhaut eines Auges zu seiner Brechkraftkorrektur so auszugestalten, daß positionsgenau, ausgehend von der Stelle, an dem das Messer an die Hornhaut angesetzt worden ist, eine in der Schnittebene des Messers liegende Tasche über einen tunnelartigen Zugang geschnitten werden kann, ohne daß mit einer Beeinträchtigung der Stabilität der Hornhaut zu rechnen ist. Außerdem soll die Vorrichtung einen weiten Bereich an Brechkraftkorrekturen zulassen.

Die Erfindung löst die gestellte Aufgabe dadurch, daß das Messer die Gestellausnehmung mit Spiel durchsetzt und daß die Halterung einen Vibrator für eine oszillierende Bewegung des Messers in der Schnittebene trägt. Durchsetzt das Messer die Gestellausnehmung mit Spiel, so kann sichergestellt werden, daß das Messer beim Verschieben, insbesondere beim Einführen des Messers in seine Schnittstellung, nicht aufliegt, so daß eine Beschädigung des Messers bzw. der Schneide des Messers ausgeschlossen werden kann. Es ist daher möglich, vergleichsweise harte Materialien für das Messer zu verwenden, da ja die Sprödigkeit dieser meist sehr harten Materialien außer Acht gelassen werden kann. Im Gegensatz zu der US 2001/0004702 A1 muß daher nicht mit einem Bruch bzw. einer Beeinträchtigung der Schneide eines Messers gerechnet werden, da das Messer mit seinen empfindlichen Teilen berührungsfrei geführt ist, wodurch erfindungsgemäß Messer mit hoher Schneidfähigkeit und kleinen Abmessungen verwendet werden können. Gerade beim Durchdringen der äußeren Schicht der Hornhaut zum Erstellen eines tunnelartigen Zugangs kann so auch davon ausgegangen werden, daß der tunnelartige Zugang äußerst präzise an die Stelle anschließt, an der zuvor das Messer an die Hornhaut angesetzt worden ist. Erleichtert wird das Eindringen des Messers dadurch, daß die Halterung einem Vibrator für eine oszillierende Bewegung des Messers in der Schnittebene trägt. Denn damit kann insbesondere die Elastizität der äußeren Schicht der Hornhaut überwunden werden, ohne mit einem erhöhten Eindrücken der Hornhautoberfläche rechnen zu müssen. Außerdem wird durch den Vibrator der Schneidvorgang annähernd kraftfrei, so daß selbst bei extrem scharfen Messern auftretende, eine der Messerbewegung zuzuordnende, Gewebsbewegung infolge der Elastizität und Zähigkeit des Gewebes ausgeschlossen werden kann. Damit kann gegenüber dem Stand der Technik ein besonders gezogener Schnitt für eine hohe Schnittgenauigkeit sichergestellt werden.

Ist die Aufnahme als Wechselaufnahme zum anschlagbegrenzten Aufnehmen von Stempeln mit unterschiedlich gekrümmten Aufsetzflächen zur Hornhautbeaufschlagung ausgebildet, wobei die Aufsetzflächen in aufeinanderfolgenden Schnitten Taschen zum Begrenzen eines linsenförmigen Gewebekörpers bestimmen, so ist es möglich, ausgehend von einem tunnelartigen Zugang ein Gewebevolumen auszuschneiden, ohne daß hierfür eine Hornhautkappe eingeschnitten werden muß. Der ausgeschnittene linsenförmige Gewebekörper kann anschließend über den tunnelartigen Zugang herausgezogen werden, wodurch es erfindungsgemäß möglich wird, einen definierten Hohlraum in der Hornhaut zu schaffen, und dabei verglichen zu den anderen Verfahren unwesentlich die Stabilität der Hornhaut zu beeinträchtigen. Dadurch ist es möglich eine Gewebslinse in der Hornhaut zu erstellen, womit eine Fehlsichtigkeit korrigiert werden kann.

Sind die Normalabstände der Umfangskanten der Aufsetzflächen der nacheinander in die Aufnahme eingesetzten Stempel zu der Schnittebene des Messers verschieden, so wird damit das Ausschneiden eines linsenförmigen Gewebekörpers erleichtert. Zwar bedingt diese Differenz der Normalabstände, daß in das Hornhautgewebe zwei tunnelartige Zugänge eingebracht werden, jedoch hat dies den Vorteil, daß die beiden Taschen in ihrer gemeinsamen Schnittlinie vorstehend sind, und so ein nur teilweises Ausschneiden des Gewebevolumens ausgeschlossen werden kann.

Bildet das nichtmetallische Messer eine Einstichspitze aus, so wird damit das Eindringen des Messers von seinem Ansatz an der Hornhaut aus erleichtert. Weist das Messer zusätzlich eine der Einstichspitze zulaufende Schneide auf, so kann mit einem zum Ringkörper radialen Verstellen des Messers ein besonders schmaler tunnelartiger Zugang in die Hornhaut geschaffen werden, was für die Stabilität der Hornhaut dienlich ist.

Besonders vorteilhafte Schnitteigenschaften des Messers werden dadurch geschaffen, daß das Messer als zweischneidiges Stichmesser ausgebildet ist, sowie vorzugsweise aus Diamantmaterial besteht, wobei das Messer eine maximale Breite von 2 mm, eine maximale Dicke von 200 µm sowie eine Klingenlänge von wenigstens 8 mm vorzugsweise 10 mm aufweist.

Besteht der Stempel aus transparentem Material, so wird damit dem Operateur die Möglichkeit gegeben, die Aufsetzfläche bzw. den Schnittvorgang auf einfache Weise zu beobachten. Ist zudem der Stempel als Vergrößerungslinse ausgebildet, deren Brennpunkt im Bereich der Aufsetzfläche zur Hornhautbeaufschlagung, vorzugsweise auf der Stempelsymmetrieachse liegt, so wird diese Überwachung noch weiter erleichtert.

Um die Größe der Aufsetzfläche des auf das Auge aufgesetzten Stempels möglichst genau einstellen zu können bzw. um den Schnittbereich der Tasche möglichst exakt einhalten zu können, empfiehlt es sich, wenn der transparente Stempel Markierungen auf seiner dem Auge zugewandten Seite zum Bestimmen der Größe der Aufsetzfläche des Stempels und der Schnittfläche am Auge aufweist.

Soll auf das Auswechseln der Stempel zum Erstellen eines ausgeschnittenen linsenförmigen Gewebekörpers verzichtet werden, so kann dies damit erreicht werden, daß zumindest die Aufsetzfläche des Stempels von einem verformbaren Material gebildet wird, das dann über eine Stelleinrichtung unterschiedlich formerhaltend krümmbar ist. Somit kann über die Stelleinrichtung dem in die Aufnahme eingesetzten Stempel anders gekrümmte Aufsetzflächen vorgegeben werden, um so einen linsenförmigen Gewebekörper ausschneiden zu können.

Weist die Halterung für das Messer einen zur Schnittebene des Messers normal wirkenden Stelltrieb auf, so ist es einem Operateur auf einfache Weise möglich, die Tiefe der eingeschnittenen Tasche zur Hornhautoberfläche einzustellen, da mit diesem Stelltrieb der Abstand des Messers gegenüber dem Stempel bzw. der Aufsetzfläche des Stempels am Auge eingestellt werden kann.

Die Halterung für das Messer besteht beispielsweise aus einem wenigstens zwei Hebelarme umfassenden Hebelsystem mit normal auf die Schnittebene des Messers stehenden Schwenkachsen. Nimmt ein Arm das Messer auf und ist der andere Arm am Gestell angelenkt, so kann auf einfache konstruktive Weise damit das Messer nicht nur radial zum Ringkörper verschoben sondern auch um seine Führungsebene senkrechte Achse verschwenkt werden.

Eine weitere Möglichkeit besteht, daß die Halterung für das Messer eine gabelartige Messerführung umfaßt, die möglichst spielfrei zwischen parallelen Flächen einer am Gestell, insbesondere an der Aufnahme, vorgesehene Umfangsnut geführt ist. Diese Ausgestaltungsform ist nicht nur besonders einfach in ihrer Konstruktion, sondern gibt auch dem Operateur den Spielraum vor, in dem er das Messer bewegen kann.

Um ein besonders einfaches Einsetzen bzw. Auswechseln der Stempel mit unterschiedlich gekrümmten Aufsetzflächen zu ermöglichen, wird vorgeschlagen, den Stempel mit Unterdruck in die Aufnahme anschlagbegrenzt zu halten. Hierfür muß lediglich in der Aufnahme eine Druckleitung vorgesehen werden, die eine zwischen der Ausnehmung und dem Stempel vorhandene Luft absaugt.

### Kurze Beschreibung der Zeichnung

In der Zeichnung ist die Erfindung anhand von Ausführungsbeispielen schematisch dargestellt. Es zeigen
- Fig. 1: ein Ausführungsbeispiel der erfindungsgemäßen Vorrichtung in teilgeschnittener Seitenansicht,
- Fig. 2: ein zweites Ausführungsbeispiel der erfindungsgemäßen Vorrichtung in teilsgeschnittener Seitenansicht,
- Fig. 3a bis c: Schnittführungen des Messers der Vorrichtungen nach Fig.1 oder 2 in einer Draufsicht,
- Fig. 3d: die mit den Schnittführungen aus den Fig. 3a bis 3c ausgeschnittene Tasche in einer Draufsicht,
- Fig. 4a: eine stempelbeaufschlagte Hornhaut mit einem angesetzten Messer zum Erstellen einer Tasche im Querschnitt,
- Fig. 4b: die nach Fig. 4a erstellte Tasche einer unbeaufschlagten Hornhaut im Querschnitt,
- Fig. 5: die Befestigung des Messers an der Halterung, die eine gabelartige Messerführung umfaßt,
- Fig. 6a bis c: Schnittführungen zum Ausschneiden eines linsenförmigen Gewebekörpers unter Verwendung von Stempel mit unterschiedlich gekrümmten Aufsetzflächen im Querschnitt,
- Fig. 7: eine Konstruktionsvariante eines Stempels mit einer krümmbaren Aufsetzfläche im vergrößerten Maßstab,
- Fig. 8a bis g: verschiedene Stempel für die erfindungsgemäße Vorrichtung in Seitenansicht,
- Fig. 9: einen Drauf- und Seitenansicht des erfindungsgemäßen Messers und
- Fig. 10: eine Draufsicht auf die Vorrichtung mit einem eingesetzten transparenten Stempel.

### Weg zur Ausführung der Erfindung

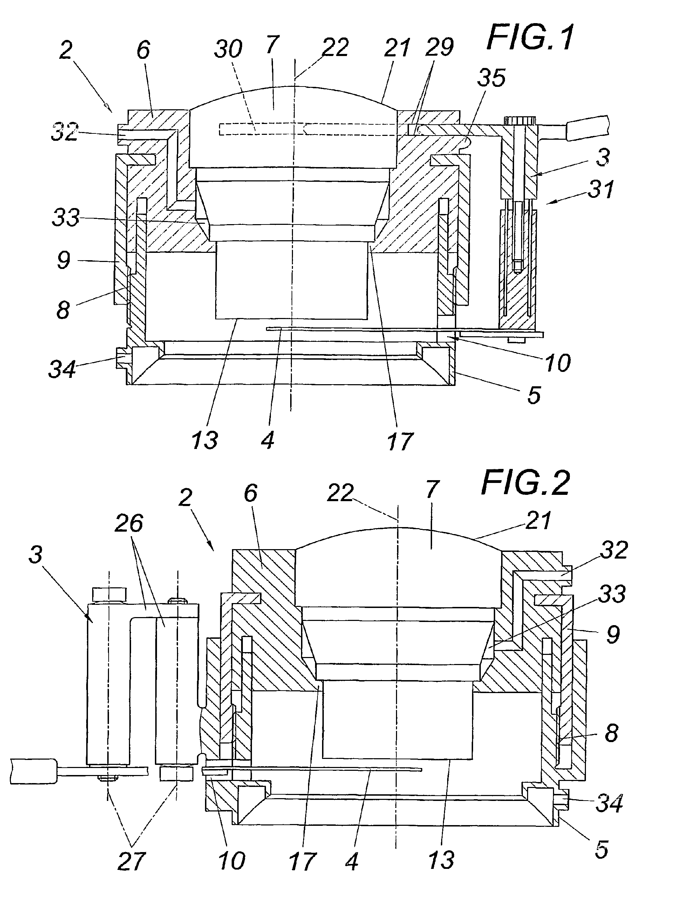

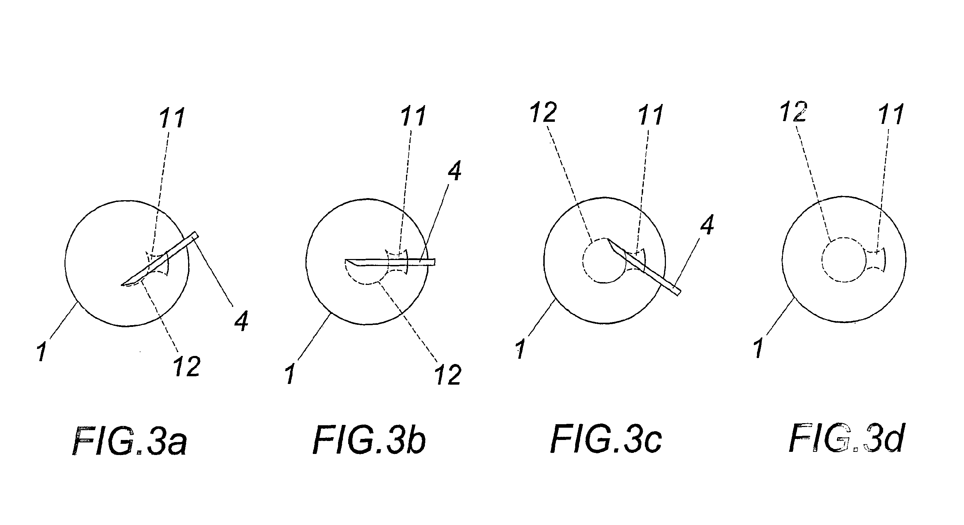

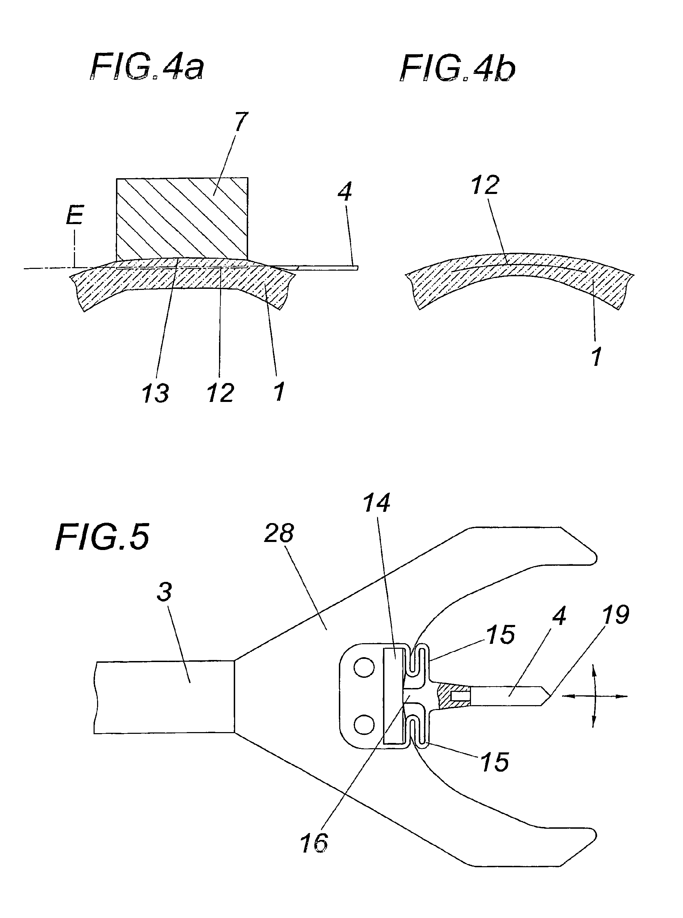

Gemäß dem Ausführungsbeispiel in Fig.1 und dem Ausführungsbeispiel in Fig. 2 umfassen die erfindungsgemäßen Vorrichtungen zum Schneiden einer Hornhaut 1 eines Auges zu einer Brechkraftkorrektur im allgemeinen ein Gestell 2 und eine Halterung 3 für ein Messer 4. Das Gestell 2 weist einen an das Auge ansaugbaren Ringkörper 5 und eine koaxial zum Ringkörper 5 verstellbare Aufnahme 6 für einen Stempel 7 zur Hornhautbeaufschlagung innerhalb des Ringkörpers 5 auf. Die Hornhaut 1 ragt also durch den Ringkörper 5 hindurch, innerhalb dessen, insbesondere höhenversetzt zum Ringkörper 5, sich der Stempel 7 zur Hornhautbeaufschlagung befindet. Zur koaxialen Verstellung ist ein Gewinde 8 am Ringkörper 5 vorgesehen, in das eine an der Aufnahme 6 drehbar gelagerte Mutter 9 eingreift. Durch Drehen der Mutter 9 kann so die Aufnahme 6 bzw. der Stempel 7 gegenüber dem Ringkörper 5 bzw. der Hornhaut 1 verstellt werden. Die Halterung 3 für das Messer 4 ist in einer auf dem Gestell 2 in einer zum Ringkörper 5 achsnormalen Ebene geführt und das Messer 4 durchsetzt das Gestell 2 in einer Umfangsausnehmung 10 mit Spiel und ist dem Stempel 7 vorgelagert. Insbesondere ist das Messer 4 durch die Halterung 3 derart geführt, daß das Messer 4 über die Halterung 3 zum Einschneiden einer lediglich einen tunnelartigen Zugang 11 aufweisenden Tasche 12 in das Hornhautgewebe einerseits radial zum Ringkörper 5 verschiebbar und andererseits um eine zur Führungsebene senkrechte Achse verschwenkbar ist, was insbesondere den Figuren 3a bis 3c zu entnehmen ist. Wobei vorstellbar ist, daß die Schnittebene E des Messers 4 auch in der Führungsebene des Messers 4 liegt. Soll nun eine Tasche 12 in die Hornhaut 1 geschnitten werden (Fig. 4a), so wird zunächst ein Stempel 7 derart auf die Hornhautoberfläche gedrückt, daß die Hornhaut 1 in definierter Weise, entsprechend der Aufsetzfläche 13 des Stempels 7 deformiert wird, was der Hornhaut 1 die Form des Stempels 7 aufprägt. Mit solch einem Drücken der Hornhaut 1 kann dann eine entsprechend große Tasche 12 in die Hornhaut 1 eingeschnitten werden. Beaufschlagt nun der Stempel 7 die Hornhaut 1 dementsprechend, so wird das Messer 4 an die Hornhaut 1 angesetzt und die äußeren Gewebsschichten der Hornhaut 1 zum Erstellen eines tunnelartigen Zugangs 11 durchgetrennt. Dabei ist es von entscheidender Bedeutung, daß das Messer 4 nicht von seinem Ansatz an der Hornhautoberfläche abgleitet. Die Erfindung weist daher einerseits ein Messer 4 auf, daß die Gestellausnehmung 10 mit Spiel durchsetzt, und andererseits trägt die Halterung 3 einen Vibrator 14 für eine oszillierende Bewegung des Messers 4 in der Schnittebene E, was der Fig. 5 entnommen werden kann. Durch das spielfreie Durchsetzten der Gestellausnehmung 10 liegt das Messer 4 nicht, wie beim Stand der Technik, auf, so daß eine Beschädigung des Messers 4, insbesondere der Schneide des Messers 4, ausgeschlossen werden kann. Deshalb sind auch besonders harte und spröde Materialien für das Messer 4 verwendbar, was aufgrund der resultierenden hohen Schärfe des Messers 4 ein Abgleiten beim Eindringen in die Hornhaut 1 so gut wie ausschließt. Es hat sich jedoch herausgestellt, daß es trotz solcher Messer 4 einen hohen Kraftaufwand bedarf, die äußere Schicht der Hornhaut 1 zu durchdringen, was erfindungsgemäß durch eine oszillierende Bewegung des Messers 4 erleichtert wird. Die Halterung 3 weist hierfür vorzugsweise einen als Piezo-Element ausgebildeten Vibrator 14 auf, der in der Schnittebene E des Messers 4 vibriert und damit das über Federn 15 am Halter 3 gehaltene Messer 4 mit Hilfe eines am Piezo-Element anliegenden Stegs 16 beaufschlagt. Vorstellbar ist jedoch, anstatt des Piezo-Elements einen Unwuchtmotor zu verwenden. Ist der tunnelartige Zugang 11 durch die äußeren Gewebsschichten der Hornhaut 1 geschnitten, dann wird das Messer zum Einschneiden einer Tasche 12 derart geführt, daß keine weiteren Verbindungen zur Hornhautoberfläche entstehen. Es wird also lediglich innerhalb der Hornhaut 1 eine Tasche 12 über den tunnelartigen Zugang 11 geschnitten, welches Schneiden selbstverständlich auch durch den Vibrator 14 erleichtert wird bzw. auch sehr genau durchgeführt werden kann. Die Tasche 12 und der tunnelartige Zugang 11 können durch ein Spreizen mit einem geeigneten Instrument ausgedehnt werden, wodurch die Einbringung von Implantaten erleichtert werden kann. Durch die geschaffene, tunnelförmige Öffnung 11 kann in die Hornhaut 1 mittels einer weiteren Vorrichtung ein vorzugsweise faltbares oder verformbares Implantat in das Implantationsbett eingebracht werden. Das Implantat entfaltet sich dann in der Tasche 12 in die gewünschte Form.

Aufgrund eines erfindungsgemäßen Schaffens einer sehr exakten Tasche 12 innerhalb einer Hornhaut 1, ist es unter anderem möglich, eine Implantation von bezüglich des Krümmungsradius standardisierten Linsen im Bereich des optischen Zentrums zu gestatten. Dies ist bedeutsam, da die natürliche Hornhautkrümmung ca. zwischen 7 mm und 9 mm variiert und bei einer ebenen, nicht gekrümmten Beaufschlagung der Hornhaut 1 einen definierter Krümmungsradius der Tasche 12 nicht erreicht kann. Durch Verwendung eines Stempels 7 mit einer bestimmten Krümmung der Aufsetzfläche 13 und der Festlegung einer bestimmten Schnittiefe, kann der Krümmungsradius der zu erstellenden Tasche 12 exakt festgelegt werden, was erlaubt, bei allen Patienten bezüglich der Krümmung der Grundfläche standardisierte Linse zu implantieren, was zu erheblichen Kosteneinsparungen in der Linsenfertigung führt.

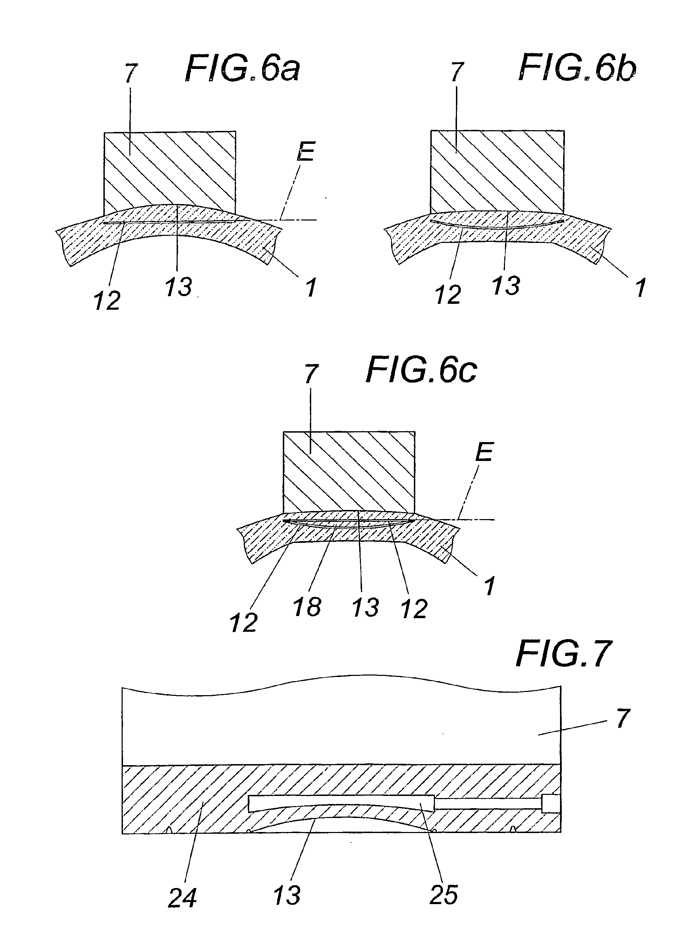

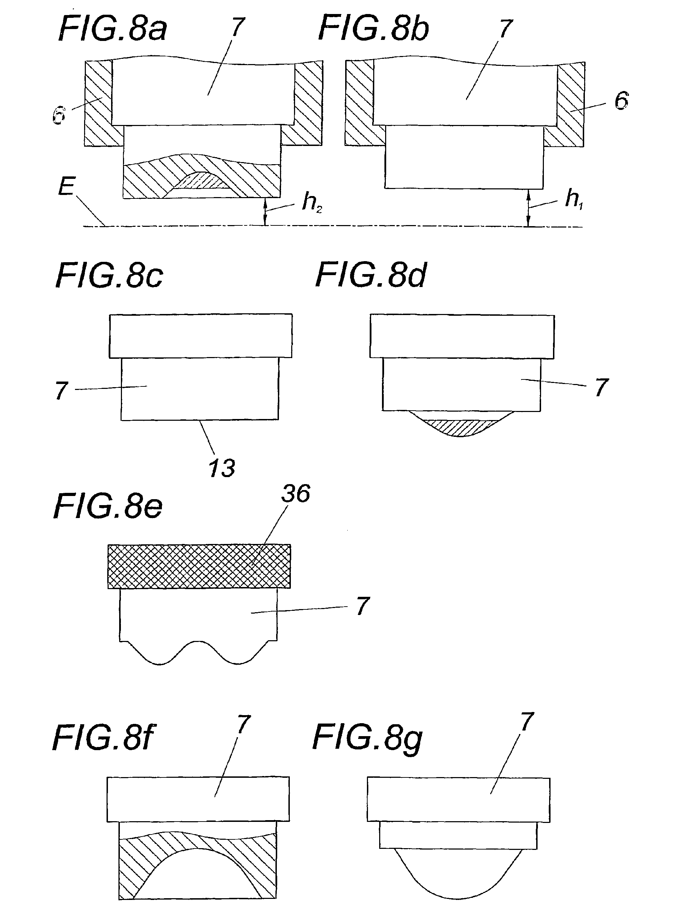

Die Aufnahme 6 für den Stempel 7 ist als Wechselaufnahme zum anschlagbegrenzten Aufnehmen von Stempeln 7 mit unterschiedlich gekrümmten Aufsetzflächen 13 zur Hornhautbeaufschlagung ausgebildet. Insbesondere den Figuren 1 und 2 ist dieser Anschlag 17 zu ersehen. Damit können auf einfache Weise in aufeinanderfolgenden Schnitten Taschen 12 zum Begrenzen eines linsenförmigen Gewebekörpers 18 bestimmt werden. Insbesondere aber, da das Gestell 2 beim Stempelwechsel vom Auge nicht abgenommen werden muß, kann mit einer Wechselaufnahme sichergestellt werden, daß immer ein in seiner Größe vorbestimmter linsenförmiger Gewebekörper 18 ausgeschnitten wird, was bei den bekannten Vorrichtungen nicht der Fall ist. Der linsenförmige Gewebekörper 18 kann gemäß den Figuren 6a bis 6c erstellt werden. Nach dem Einschneiden einer ersten Tasche 12 in das Hornhautgewebe, wie auch vorstehend beschrieben, wird nach dem Entfernen des Messers 4 aus dem Hornhautgewebe (Fig. 6a) eine zweite Tasche 12 - geprägt von einem weiteren Stempel 7 mit einer unterschiedlich gekrümmten Aufsetzfläche 13- eingeschnitten (Fig. 6b und c). Der Stempel 7 muß dafür entweder ausgewechselt oder bezüglich seiner Aufsetzfläche 13 verändert werden, wie dies beispielsweise mit einem Stempel 7 gemäß Fig. 7 möglich ist. Durch einen erneuten Schnitt durch den tunnelartigen Zugang 11 entsteht neben einer neuen Tasche 12 auch ein ausgeschnittener linsenförmiger Gewebekörper 18 (Fig. 6c), der beispielsweise mit einer Pinzette durch den tunnelartigen Zugang 11 herausgezogen werden kann. Der linsenförmige Gewebekörper entspricht in dessen Form der Differenz der unterschiedlich gekrümmten Aufsetzflächen 13 der beiden Stempel 7 bzw. der gewünschten Dioptrienänderung durch entsprechende Veränderung der vorderen Hornhautkrümmung. Außerdem weist der Stempel 7 in seinem oberen Bereich eine oberflächengravierte Grifffläche 36 auf (Fig. 8e), damit ein einfaches Wechseln des Stempels 7 möglich wird. Dabei ist es dienlich, daß der Stempel 7 der Aufnahme 6 vorragt, um so einfach gefaßt werden zu können.

Um das Ausschneiden eines linsenförmigen Gewebekörpers 18 zu erleichtern, kann das Messer 4 für den zweiten Schnitt geringfügig tiefer in die Hornhaut 1 einschneiden (Fig. 8a und 8b). Dies kann dadurch erreicht werden, daß die Normalabstände h1 bzw. h2 der Umfangskanten der Aufsetzflächen 13 der nacheinander in die Aufnahme 6 eingesetzten Stempeln 7 zu der Schnittebene E des Messers 4 verschieden sind. Ausführungsbeispiele für verschiedene Aufsetzflächen 13 von Stempeln 7 finden sich in den Fig. 8a bis g wieder. Die Abmessungen des mit den Stempeln nach Fig. 8a und 8b sowie 8c und 8d herausgeschnittenen linsenförmigen Gewebekörpers 18 ist schraffiert angedeutet.

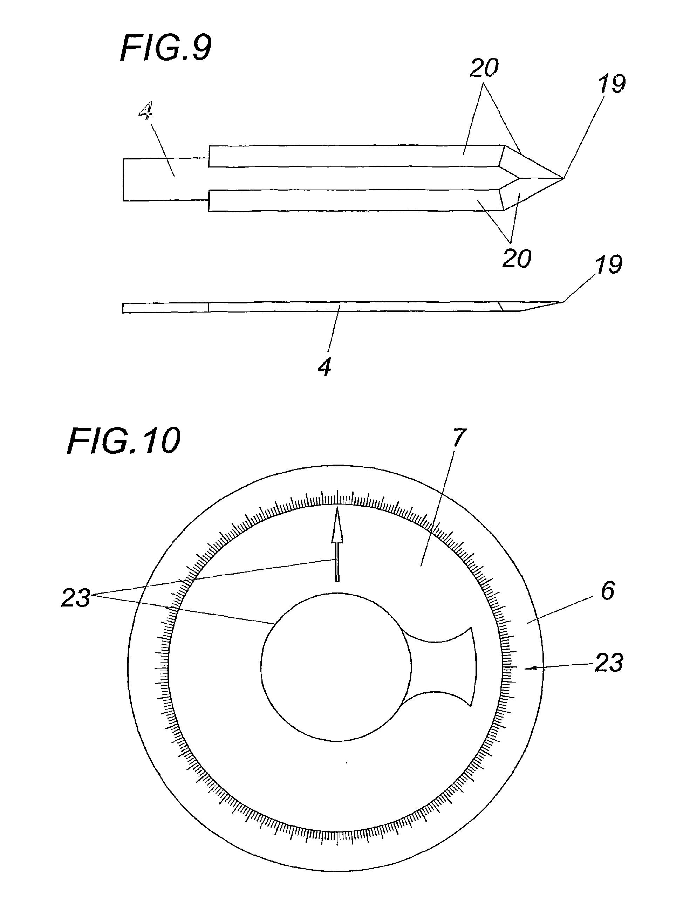

Das vorzugsweise nichtmetallische Messer 4 bildet nach Fig. 9 eine Einstichspitze 19 mit zwei von dieser Spitze ausgehenden Schneiden 20 aus. Gerade eine Einstichspitze 19 hat sich besonders zum Durchdringen der äußeren Schicht der Hornhaut 1 bzw. zum Erstellen eines tunnelartigen Zugangs 11 bewährt. Die Form des Messers 4 ähnelt der eines zweischneidigen Stichmessers. Besondere vorteilhafte Schnittverhältnisse ergeben sich, wenn das Messer 4 aus einem Diamantmaterial mit einer maximalen Breite von 2mm, eine maximale Dicke von 200µm sowie eine Klingenlänge von wenigstens 8mm, vorzugsweise von 10mm, besteht.

Die Stempel 7 bestehen vorzugsweise aus transparentem Material, wie Kunststoff oder Glas, und sind wie in Fig. 1 und 2 als Vergrößerungslinsen 21 ausgebildet, deren Brennpunkt im Bereich der Aufsetzfläche 13, vorzugsweise auf der Stempelsymmetrieachse 22 liegt. Mit einer solchen Ausbildung eines Stempels 7 ist es einem Operateur vergleichsweise einfach möglich den Fortschritt der Behandlung der Hornhaut 1 zu überwachen.

Der transparente Stempel 7 weist auf der dem Auge zugewandten Seite Markierungen 23 auf. Diese Markierungen 23 erlauben es einem Operateur beispielsweise sich hinsichtlich des Ansatzes des Messers 4 für einen tunnelartigen Zugang bestmöglich zu orientieren. Auch können Markierungen 23 für die optische Behandlungszone angebracht, um so einem Operateur die Grenzen der einzuschneidenden Tasche 12 anzugeben. Außerdem weist der transparente Stempel 7 Markierungen 23 an seiner dem Auge abgewandten Seite auf, die in Zusammenhang mit Markierungen 23 an der Aufnahme 6 für den Stempel 7 stehen (Fig. 10). Damit wird es dem Operateur möglich durch einen Verdrehversatz des Stempels 7 zur Aufnahme 6 Brechkraftkorrekturen, insbesondere bei Astigmatismus, durchzuführen.

Die Aufsetzfläche 13 des in Fig. 7 dargestellten Stempels besteht aus einem verformbaren Material 24, das über eine Stelleinrichtung 25 unterschiedlich formerhaltend, krümmbar ist. Über einen Anschluß an die Stelleinrichtung 25 kann der Hohlraum der Stelleinrichtung 25 mit Druckgas und Druckflüssigkeit oder dgl. beaufschlagt, gemäß der Beaufschlagung der Stempel 7 seine Aufsetzfläche 13 unterschiedlich formerhaltend krümmt.

Nach dem Ausführungsbeispiel gemäß Fig. 2 besteht die Halterung 3 aus wenigstens zwei Hebelarmen 26 umfassenden Hebelsystem mit normal auf die Schnittebene E des Messers 4 stehenden Schwenkachsen 27, wobei ein Hebelarm 26 das Messer 4 aufnimmt und der andere Hebelarm 26 am Gestell 2, vorzugsweise an der Aufnahme 6, angelenkt ist.

Gemäß dem Ausführungsbeispiel nach Fig. 1 kann die Halterung 3 auch eine das Messer 4 aufnehmende gabelartige Messerführung 28 umfassen, die möglichst spielfrei zwischen parallelen Flächen 29 einer am Gestell 2, insbesondere an der Aufnahme 6, vorgesehenen Umfangsnut 30 geführt ist. Das Messer 4 ist versetzt zur gabelartigen Messerführung 28 angeordnet, wobei der Abstand zwischen Schnittebene E des Messers 4 und Aufsetzfläche 13 des Stempels 7 mittels eines Stelltriebs 31 in der Form eines Schraubentriebes eingestellt werden kann. Damit die gabelartig Messerführung 28 einfach eingeschoben werden kann, ist an der Aufnahme 6 eine Noppe 35 vorgesehen.

Der Stempel 7 ist in der Aufnahme 6 mittels Unterdruck fixierbar. Dazu kann über eine Leitung 32 Luft aus einer Kammer zwischen Aufnahme und Stempel abgesaugt werden. Der Stempel ist hiefür in der Form eines Kegelstumpfs ausgebildet, was ein einfaches Einsetzten des Stempels erlaubt. Weiters ist vorstellbar, daß statt der Druckleitung andere mechanische Halter verwendet werden, wie z.B. Bajonettverschluß, magnetische, elektromagnetische, hydraulische oder andere gleichwertige Mechanismen. Ähnlich wird dies mit einem Ansaugen des Ringkörpers 5 an das Auge durch eine Druckleitung 34 durchgeführt.

## Patentansprüche

1. Vorrichtung zum Schneiden der Hornhaut (1) eines Auges zu seiner Brechkraftkorrektur mit einem Gestell (2), das sowohl einen an das Auge ansaugbaren Ringkörper (5) als auch eine koaxial zum Ringkörper (5) verstellbare Aufnahme (6) für einen Stempel (7) zur Hornhautbeaufschlagung innerhalb des Ringkörpers (5) aufweist, und mit einer auf dem Gestell (2) in einer zum Ringkörper (5) achsnormalen Ebene geführten Halterung (3) für ein das Gestell (2) in einer Umfangsausnehmung (10) durchsetzendes, dem Stempel (7) vorgelagertes Messer (4), das über die Halterung (3) zum Einschneiden einer lediglich einen tunnelartigen Zugang (11) aufweisenden Tasche (12) in das Hornhautgewebe einerseits radial zum Ringkörper (5) verschiebbar und andererseits um eine zur Führungsebene senkrechte Achse verschwenkbar ist, **dadurch gekennzeichnet, daß** das Messer (4) die Gestellausnehmung (10) mit Spiel durchsetzt und daß die Halterung (3) einen Vibrator (14) für eine oszillierende Bewegung des Messers (4) in der Schnittebene (E) trägt.

2. Vorrichtung nach Anspruch 1, **dadurch gekennzeichnet, daß** die Aufnahme (6) als Wechselaufnahme zum anschlagbegrenzten Aufnehmen von Stempeln (7) mit unterschiedlich gekrümmten Aufsetzflächen (13) zur Hornhautbeaufschlagung ausgebildet ist, wobei die Aufsetzflächen (13) in aufeinanderfolgenden Schnitten Taschen (12) zum Begrenzen eines linsenförmigen Gewebekörpers (18) bestimmen.

3. Vorrichtung nach Anspruch 1 oder 2, **dadurch gekennzeichnet, daß** die Normalabstände der Umfangskanten der Aufsetzflächen (13) der nacheinander in die Aufnahme (6) eingesetzten Stempeln (7) zu der Schnittebene (E) des Messers (4) verschieden sind.

4. Vorrichtung nach Anspruch 1, 2 oder 3, **dadurch gekennzeichnet, daß** das nichtmetallische Messer (4) eine Einstichspitze (19) und zumindest eine der Einstichspitze zulaufende Schneide (20) bildet.

5. Vorrichtung nach Anspruch 4, **dadurch gekennzeichnet, daß** das als zweischneidiges Stichmesser ausgebildete Diamantmesser eine maximale Breite von 2mm, eine maximale Dicke von 200µm sowie eine Klingenlänge von wenigstens 8mm, vorzugsweise von 10mm, aufweist.

6. Vorrichtung nach einem der Ansprüche 1 bis 5, **dadurch gekennzeichnet, daß** der aus transparentem Material bestehende Stempel (7) als Vergrößerungslinse (21) ausgebildet ist, deren Brennpunkt im Bereich der Aufsetzfläche (13) zur Hornhautbeaufschlagung, vorzugsweise auf der Stempelsymmetrieachse (22) liegt.

7. Vorrichtung nach Anspruch 6, **dadurch gekennzeichnet, daß** der transparente Stempel (7) Markierungen (23) sowohl auf seiner dem Auge zugewandten Seite zum Bestimmen der Größe der Aufsetzfläche (13) des Stempels am Auge als auch auf seiner dem Auge gegenüberliegenden Seite zum Bestimmen des Verdrehversatzes des Stempels (7) zur Aufnahme (6) aufweist.

8. Vorrichtung nach einem der Ansprüche 1 bis 7, **dadurch gekennzeichnet, daß** zumindest die Aufsetzfläche (13) des Stempels (7) von einem verformbaren Material (24) gebildet wird, das über eine Stelleinrichtung (25) unterschiedlich formerhaltend, krümmbar ist.

9. Vorrichtung nach einem der Ansprüche 1 bis 8, **dadurch gekennzeichnet, daß** die Halterung (3) für das Messer (4) einen zur Schnittebene (E) des Messers (4) normal wirkenden Stelltrieb (31) zum Einstellen des Abstands des Messers (4) gegenüber dem Stempel (7) aufweist.

10. Vorrichtung nach einem der Ansprüche 1 bis 9, **dadurch gekennzeichnet, daß** die Halterung (3) aus einem wenigstens zwei Hebelarme (26) umfassenden Hebelsystem mit normal auf die Schnittebene (E) des Messers (4) stehenden Schwenkachsen (27) besteht, wobei ein Arm das Messer (4) aufnimmt und der andere Arm am Gestell (2) angelenkt ist.

11. Vorrichtung nach einem der Ansprüche 1 bis 10, **dadurch gekennzeichnet, daß** die Halterung (3) eine das Messer (4) aufnehmende gabelartige Messerführung (28) umfaßt, die möglichst spielfrei zwischen parallelen Flächen (29) einer am Gestell (2), insbesondere an der Aufnahme (6), vorgesehenen Umfangsnut (30) geführt ist.

12. Vorrichtung nach einem der Ansprüche 1 bis 11, **dadurch gekennzeichnet, daß** der Stempel (7) mit Unterdruck in der Aufnahme (6) anschlagbegrenzt gehalten ist.

## Claims

1. A device for cutting the cornea (1) of an eye in order to correct the refractive power thereof, having a frame (2) that comprises a fixation ring (5), which may be drawn onto the eye, as well as a receptacle (6), which may be coaxially displaced relative to the fixation ring (5) and which serves to accommodate an applanator (7) for deforming the cornea within the fixation ring (5), and a holding device (3), which is guided on the frame (2) in a plane that is perpendicular to the axis with regard to the fixation ring (5) and which serves to hold a blade (4), which passes through the frame (2) via a peripheral recess (10) and which is mounted in front of the applanator (7), being radially displaceable relative to the fixation ring (5) as well as movable around an axis perpendicular to the guiding plane via the holding device (3), for the purpose of cutting a pocket (12) through a merely tunnel-like entry (11) into the corneal tissue, **characterized in that** said blade (4) passes through the frame recess (10) with clearance and the holding device (3) supports a vibrator (14) for setting the blade (4) in oscillatory motion in the cutting plane (E).

2. The device according to Claim 1, **characterized in that** said receptacle (6) is designed as a changeover-receptacle for the stop-delimited receipt of interchangeable applanators (7) having differently curved contact faces (13) for applanation of the cornea, the contact faces (13) in sequential incisions determining pockets (12) for delimiting a lens-shaped portion of tissue (18).

3. The device according to Claim 1 or 2, **characterized in that** said perpendicular distances of the peripheral edges of the contact faces (13) of the applanators (7) sequentially inserted in the receptacle (6) are different with respect to the cutting plane (E) of the blade (4).

4. The device according to Claim 1, 2 or 3, **characterized in that** said non-metallic blade (4) forms a pointed tip (19) and at least one cutting edge (20) extending towards the tip.

5. The device according to Claim 4, **characterized in that** said diamond blade, which is designed as a double-edged knife, has a maximum width of 2 mm, a maximum thickness of 200 microns and a blade length of at least 8 mm, preferably 10 mm.

6. The device according to one of the Claims 1 to 5, **characterized in that** said applanator (7), being made of transparent material, is designed as an enlargement lens (21), the focal point of which lies in the area of the contact face (13) for applanation of the cornea, preferably on the axis of symmetry of the applanator (22).

7. The device according to Claim 6, **characterized in that** said applanator (7) has markings (23) on its side facing the eye, for determining the size of the contact face (13) of the applanator on the eye, as well as on its side diametrically opposite the eye, for determining the offset of the applanator (7) in relation to the receptacle (6).

8. The device according to one of the Claims 1 to 7,
**characterized in that** at least the contact face (13) of the applanator (7) is made of a deformable material (24), which may be curved via an actuator (25) to obtain different shapes that are maintained.

9. The device according to one of the Claims 1 to 8, **characterized in that** said holding device (3) supporting the blade (4) has a position adjuster (31) operating perpendicular to the cutting plane (E) of the blade (4) for adjusting the distance of the blade (4) from the applanator (7).

10. The device according to one of the Claims 1 to 9,
**characterized in that** said holding device (3) consists of a lever system comprising at least two lever arms (26), having pivot axes (27) which are perpendicular to the cutting plane (E) of the blade (4), one arm receiving the blade (4) and the other arm being linked to the frame (2).

11. The device according to one of the Claims 1 to 10, **characterized in that** said holding device (3) comprises a forked blade guide (28) for receiving the blade (4), which is guided with minimal clearance between parallel faces (29) of a peripheral groove (30) provided on the frame (2), in particular on the receptacle (6).

12. The device according to one of the Claims 1 to 11, **characterized in that** said applanator (7) is held in the receptacle (6) in a stop-delimited position by using a vacuum.

## Revendications

1. Dispositif pour inciser la cornée (1) d'un oeil en vue de corriger sa réfringence, avec un bâti (2) comprenant un élément annulaire (5) pouvant être fixé par succion sur l'oeil et un réceptacle (6) mobile dans le sens coaxial par rapport à l'élément annulaire (5) pour un élément pressant (7) sur la cornée à l'intérieur de l'élément annulaire (5), et avec une monture (3) guidée sur le bâti (2) dans un plan perpendiculaire à l'axe de l'élément annulaire (5) pour un scalpel (4) traversant le bâti (2) dans une ouverture sur la circonférence (10) et monté à l'avant de élément pressant (7), qui est d'une part capable de translation sur la monture (3), dans le sens radial par rapport à l'élément annulaire (5), pour inciser une poche (12) du tissu cornéen présentant seulement un accès (11) en forme de tunnel, et d'autre part autour capable de pivotement autour d'un axe perpendiculaire au plan de guidage, **caractérisé en ce que** le scalpel (4) traverse l'ouverture dans le bâti (10) avec une distance et **en ce que** la monture (3) porte un vibrateur (14) réalisant un mouvement oscillant du scalpel (4) dans le plan de coupe (E).

2. Dispositif selon la revendication 1, **caractérisé en ce que** le réceptacle (6) est conformé comme un réceptacle interchangeable pouvant recevoir avec une butée de limitation des éléments pressants (7) ayant des surfaces d'application (13) de courbure différente pour agir sur la cornée, les surfaces d'application (13) formant par étapes successives des poches (12) pour délimiter un élément tissulaire en forme de lentille (18).

3. Dispositif selon la revendication 1 ou 2, **caractérisé en ce que** les distances euclidiennes des bords périphériques des surfaces d'application (13) des éléments pressants (7) introduits l'un après l'autre dans le réceptacle (6) par rapport au plan de coupe (E) du scalpel (4) sont différentes.

4. Dispositif selon la revendication 1, 2 ou 3, **caractérisé en ce que** le scalpel (4) non métallique forme une pointe de ponction (19) et au moins un tranchant (20) se rétrécissant vers une pointe de ponction.

5. Dispositif selon la revendication 4, **caractérisé en ce que** la lame diamantée conçue comme un scalpel de ponction à double tranchant a une largeur maximale de 2 mm, une épaisseur maximale de 200 µm et une longueur de lame d'au moins 8 mm, de préférence de 10 mm.

6. Dispositif selon l'une des revendications 1 à 5, **caractérisé en ce que** l'élément pressant (7) fait de matériau transparent est conformé comme une lentille grossissante (21) dont la focale se trouve au niveau de l'axe d'application (13) pour agir sur la cornée, de préférence sur l'axe de symétrie (22) du poinçon.

7. Dispositif selon la revendication 6, **caractérisé en ce que** l'élément pressant transparent (7) présente des repères (23) sur son côté orienté vers l'oeil en vue de déterminer la taille de la surface d'application (13) de l'élément pressant sur l'oeil ainsi que sur son côté opposé à l'oeil pour déterminer le décalage par rotation de l'élément pressant (7) par rapport au réceptacle (6).

8. Dispositif selon l'une des revendications 1 à 7, **caractérisé en ce que** la surface d'application (13) de l'element pressant (7) au moins est faite d'un matériau déformable (24), qui peut être courbé selon différentes formes par un dispositif d'ajustement (25).

9. Dispositif selon l'une des revendications 1 à 8, **caractérisé en ce que** la monture (3) pour le scalpel (4) possède un entraînement d'ajustement (31) agissant perpendiculairement au plan de coupe (E) du scalpel (4) pour régler la distance entre le scalpel (4) et l'élément pressant (7).

10. Dispositif selon l'une des revendications 1 à 9, **caractérisé en ce que** la monture (3) se compose d'un système de leviers comportant au moins deux bras de levier (26) avec des axes de pivotement (27) perpendiculaires au plan de coupe (E) du scalpel (4), un bras recevant le scalpel (4) et l'autre bras étant articulé sur le bâti (2).

11. Dispositif selon l'une des revendications 1 à 10, **caractérisé en ce que** la monture (3) comprend un guide-lame (28) en forme de fourche recevant le scalpel (4), qui est guidé avec la moins de distance possible entre des surfaces (29) parallèles d'une gorge périphérique (30) prévue sur le bâti (2) et en particulier sur le réceptacle (6).

12. Dispositif selon l'une des revendications 1 à 11, **caractérisé en ce que** l'élément pressant (7) est retenu par une dépression dans le réceptacle (6) en étant limité par une butée.
